# EUROPEAN PATENT SPECIFICATION

(11) **EP 4 389 081 B1**
(45) Date of publication and mention of the grant of the patent: **28.10.2026**
(21) Application number: 23218189.1
(22) Date of filing: 19.12.2023
(51) Int. Cl.: A61F 2/38

(54) **CONSTRAINED PROSTHETIC KNEE**
EINGESCHRÄNKTES PROTHESENKNIE
PROTHÈSE DE GENOU CONTRAINTE

(30) Priority: 22.12.2022 US 202263434563 P; 01.12.2023 US 202318526693
(43) Date of publication of application: 26.06.2024
(73) Proprietor: Zimmer, Inc., Warsaw, IN 46580 (US)
(72) Inventor: YOKO, Tim, Granger, 46530 (US); BYRD, Brian D., North Webster, 46555 (US); BARKER, Joshua, Warsaw, 46582 (US); REIDY, John, Warsaw, 46580 (US); DYKEMA, Scott, Warsaw, 46582 (US); VERNON, Douglas Murray, Warsaw, 46580 (US)
(74) Representative: Taor, Simon Edward William

(56) References cited:
- EP-A1- 3 127 510
- US-A1- 2010 174 378

## Description

### CLAIM OF PRIORITY

This application claims the benefit of U.S. Provisional Patent Application Serial No. 63/434,563, filed on December 22, 2022, and U.S. Patent Application Serial No. 18/526,693, filed on December 1, 2023, the benefit of priority of which is claimed hereby.

### TECHNICAL FIELD

The present disclosure generally relates to orthopedic prostheses. More specifically, the present disclosure relates to orthopedic prostheses used in constrained knee arthroplasties.

### BACKGROUND

Orthopedic procedures and prostheses are commonly utilized to repair or replace damaged bone and tissue in the human body. Generally, the knee is formed by the pair of condyles at the distal portion of the femur, the lower surfaces of which bear upon the correspondingly shaped proximal surface plateau of the tibia. The femur and tibia are connected by means of ligaments such as, the posterior cruciate ligament, the lateral collateral ligament, the medial collateral ligament, and the anterior cruciate ligament. These ligaments provide stability to the knee j oint.

Prosthetic knee joints can be considered either constrained or unconstrained. Constrained prosthetic knee systems can include femoral and tibial prostheses, which are mechanically linked or constrained to each other to limit relative movement between the femoral and tibial prostheses. Common mechanisms for such mechanical linkage can include a hinge, band, or other linkage structure. An unconstrained prosthetic knee system includes femoral and tibial prostheses, which are not mechanically linked. An unconstrained knee utilizes the existing ligaments and other soft tissue of the patient to provide joint stability. Constrained prosthetic knees have particular applicability to cases where a patient has experienced ligament loss or the existing ligaments do not provide adequate support and stability to the knee.

EP3127510A1 discloses a total knee prosthesis including a tibial component having a baseplate portion and a protruding portion extending from the baseplate portion is disclosed. An axle is connectable to the tibial component directly or indirectly. A femoral component has an articular surface and an opening for receipt of the axle. The opening defines a pivot center about which the femoral component rotates. The articular surface has a posterior portion that defines a first flexion radius and a first flexion center. The first flexion center is offset from the pivot center a distance substantially equal to a product of a constant and the first flexion radius.

### OVERVIEW

This disclosure pertains generally to improved constrained knee prostheses, particularly those utilizing a hinge post. Some constrained knee prostheses with hinge posts utilize a design where the femoral component (and hinge post) are free to move generally proximal/distal relative to the tibial baseplate and the tibial bearing component. Such an arrangement can allow for distraction of the knee joint. However, the present inventors have recognized that a certain segment of patients receiving a constrained knee prosthesis with the hinge post may have insufficient soft tissue in the knee joint to prevent distraction and then luxation of the femoral component from the tibial baseplate and the tibial bearing component. Luxation can result in pain and other complications for the patient.

Design constraints of an improved prosthesis require components with increased widths to improve the strength of such components. Additionally, the inventors of the present application have discovered that present knee prostheses have minimal adjustments that can change the rotation of the femoral component as it relates to a tibial baseplate.

The invention is set out in the appended claims.

### BRIEF DESCRIPTION OF THE DRAWINGS

Various embodiments are illustrated by way of example in the figures of the accompanying drawings. Such embodiments are demonstrative and not intended to be exhaustive or exclusive embodiments of the present subject matter.
FIGS. 1 and 2 illustrate knee joint structures providing suitable environments in which the constrained prosthesis assemblies in accordance with an example of the present application can be utilized.
FIG. 3 is a perspective view of a constrained knee prosthesis assembly according to an example of the present application.
FIG. 4 is an exploded view of the constrained knee prosthesis assembly of FIG. 3.
FIG. 5 is a cross-sectional view of an example of a constrained knee prosthesis according to an example of the present application.
FIG. 6 is a perspective view of an example of a femoral component according to an example of the present application.
FIG. 7 is a perspective view of a portion of a constrained knee prosthesis according to an example of the present application.
FIG. 8 is an exploded view of the portion of the constrained knee prosthesis from FIG. 7 according to an example of the present application.
FIG. 9 is a cross-sectional view of a portion of a constrained knee prosthesis according to an example of the present application.

### DETAILED DESCRIPTION

The present application relates to a constrained knee prosthesis. The prosthesis can include a shackle with an increased width to improve the strength of the shackle. A femoral component can include frustoconical profiles to accommodate the shackle with the increased width and fit within the same overall prosthesis envelope. The prosthesis can also include walls that at least partially surround the shackle and are configured to engage with the frustoconical profiles on the femoral component that can also include frustoconical profiles that are complementary to the frustoconical profiles on the femoral component.

The prosthesis can also include a bump stop that can be removably attached to the femoral component such that when the knee joint is in an extended state, the bump stop contacts the shackle to prevent additional rotation of the femoral component related to the tibial baseplate. The prosthesis can be configured to make the bump stop serviceable. Therefore, the bump stop can be removed, adjusted, e.g., to quickly change the rotation of the femoral component related to the tibial baseplate without removing the bump stop, or replaced without removing the prosthesis from the knee. For example, the prosthesis can be configured to remove, adjust, or replace the bump stop when the knee joint is in a flexed state.

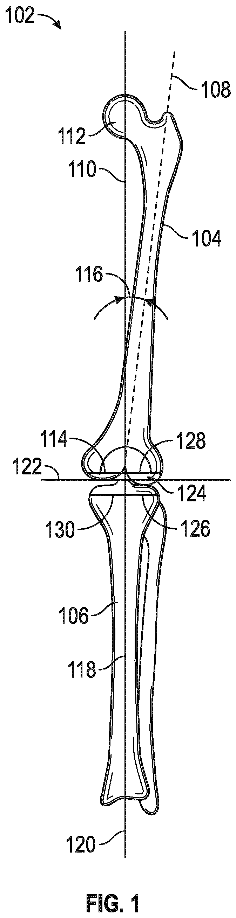

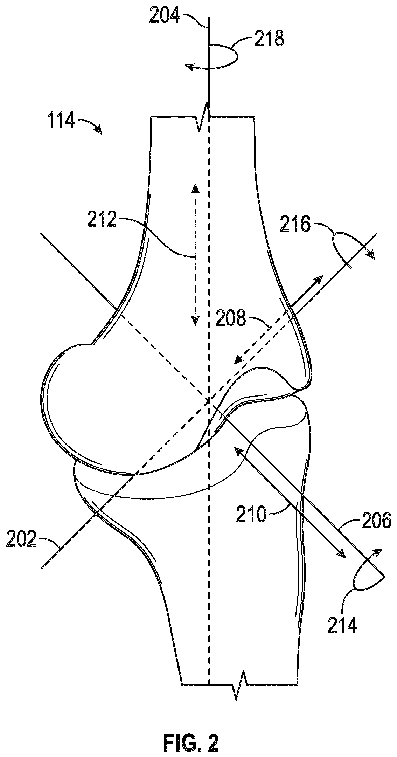

To better understand knee joint replacement procedures, it can be helpful to understand the relationship between bones and bone cuts that can be made to orient various provisional and permanent prosthesis components within a knee joint. FIGS. 1 and 2 illustrate several features of knee joint structures and orientations. In FIG. 1, a frontal view of a lower limb 102, including a femur 104 and a tibia 106, illustrates various lower limb axes. The femur 104 has an anatomic axis 108 that coincides generally with its intramedullary canal. The femur 104 also has a mechanical axis 110, or load axis, running from the center of a femoral head 112 to the center of a knee joint 114. The angle 116 extending between these two axes varies among the patient population but is generally between 5-7 degrees, inclusive. Like the femur 104, the tibia 106 also has an anatomic axis coinciding generally with its intramedullary canal. The mechanical axis 118 of the tibia 106 runs from the center of the knee joint 114 to the center of an ankle region 120 and is generally collinear with its anatomic axis.

A joint line 122, about which the knee joint 114 flexes, is approximately parallel to a line through medial and lateral femoral condyles 124 and to a tibial plateau 126. Although illustrated as perpendicular in FIG. 1, the joint line 122 can extend at a varus or valgus angle relative to the mechanical axis 110 and the mechanical axis 118 of the femur 104 and tibia 106, respectively. Normally, during a partial or total knee replacement procedure, portions of a distal end of the femur 104 or a proximal end of the tibia 106 are resected to be parallel or approximately parallel to the joint line 122, and thus perpendicular to the mechanical axis 110 and the mechanical axis118, as indicated at 128 and 130, respectively.

Typical knees can move between an extended state, where a longitudinal axis of the femur 104 and a longitudinal axis of the tibia 106 are essentially parallel, and a flexed state, where the longitudinal axis of the femur 104 and the longitudinal axis of the tibia 106 are angled relative to each other around 140 degrees. In examples, the extended state can be restricted to +/- 10 degrees, and the flexed state can be much less than 140 degrees. For example, the flexed state can include limits where the longitudinal axis of the femur 104 and the longitudinal axis of the tibia 106 are angled relative to each other around 90 to 140 degrees.

FIG. 2 illustrates a closer view of the knee joint 114 and its coordinate system, in which a medial/lateral axis 202 corresponds approximately to the joint line 122 (FIG. 1), a proximal/distal axis 204 corresponds approximately to the mechanical axis 110 (FIG. 1), the mechanical axis 118 (FIG. 1), and an anterior/posterior axis 206 is approximately normal to the other two axes. The arrows can depict positions along each of these axes, representing the medial/lateral 208, anterior/posterior 210, and proximal/distal 212 positioning of inserted prosthesis components. Rotation about each of these axes can also be depicted by arrows. Rotation about the proximal/distal axis 204 can correspond anatomically to external rotation of a femoral component, while rotation about the anterior/posterior axis 206 and medial/lateral axis 202 can correspond to extension plane slope and varus/valgus angle of a component, respectively. Depending on a position of the proximal tibial cut 130 (FIG. 1) made, a varus/valgus angle 214, extension plane angle 216, external rotation 218, or joint extension gap can be affected. Similarly, a position of the distal femoral cut 128 (FIG. 1) can affect the location of the joint line 122, the extension gap, the varus/valgus angle 214, or the extension plane angle 216.

As used herein, the terms "proximal" and "distal" should be given their generally understood anatomical interpretation. The term "proximal" refers to a direction generally toward the torso of a patient, and "distal" refers to the opposite direction of proximal, i.e., away from the torso of a patient. It should be understood that the terms "proximal" and "distal" should be interpreted as though the patient were standing with the knee joint in extension. The intent is to differentiate the terms "proximal" and "distal" from the terms "anterior" and "posterior." As used herein, the terms "anterior" and "posterior" should be given their generally understood anatomical interpretation. Thus, "posterior" refers to a rear of the patient, e.g., a back of the knee. Similarly, "anterior" refers to a front of the patient, e.g., a front of the knee. Thus, "posterior" refers to the opposite direction of "anterior." Similarly, the term "lateral" refers to the opposite direction of "medial." The term "medial/lateral" means medial to lateral or lateral to medial. The term "proximal/distal" means proximal to distal or distal to proximal. The term "anterior/posterior" means anterior to posterior or posterior to anterior.

As used herein, the "periphery" of a tibial baseplate refers to any periphery as viewed in a top plan view, e.g., in a generally transverse anatomical plane. Alternatively, the periphery of a tibial baseplate may be any periphery as viewed in the bottom plan view, e.g., in a generally transverse plane and looking at the distal surface adapted to contact a resected proximal surface of a tibial bone.

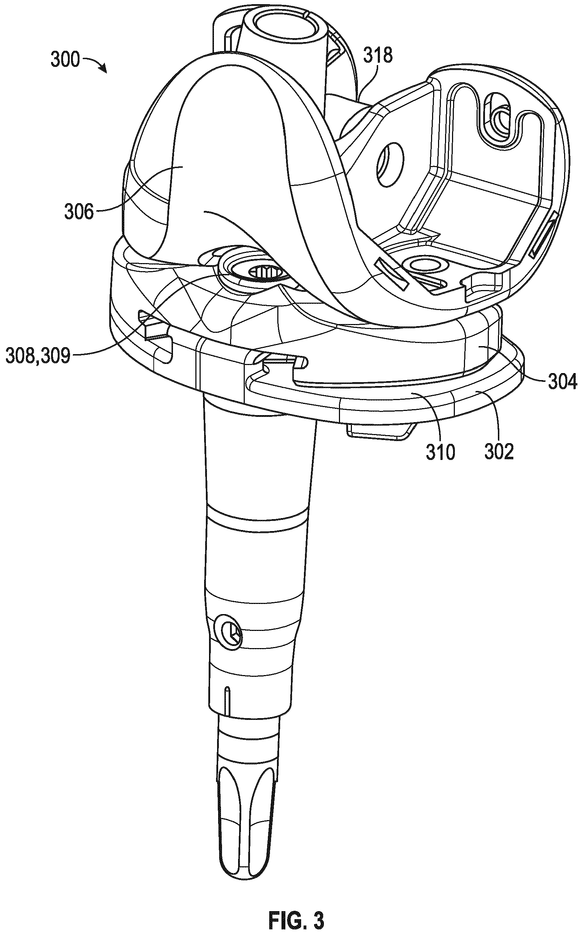

FIG. 3 shows a prosthesis assembly 300 for a constrained knee. The prosthesis assembly 300 includes a tibial baseplate 302, a tibial bearing component 304 (sometime called a meniscal component, poly, articular component or bearing), a femoral component 306 and a hinge post 308.

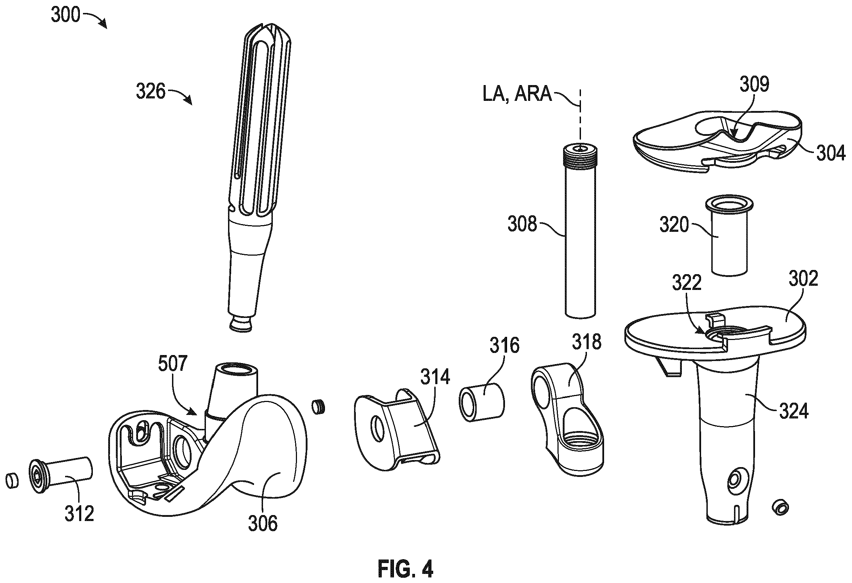

The tibial bearing component 304 can be coupled to and can be positioned atop a proximal surface 310 of the tibial baseplate 302. The tibial bearing component 304 can be formed of polymer material such as Ultra-High-Molecular-Weight-Polyethylene ("UHMWPE"), or the like. The tibial bearing component 304 can be configured to articulate with the femoral component 306 through knee joint flexion and extension as known in the art. The prosthesis assembly 300 has the femoral component 306 and the tibial baseplate 302 mechanically linked to one another. This is accomplished by the hinge post 308 and other components further illustrated and discussed in FIG. 4. The hinge post 308 is coupled to the femoral component 306 and is received within a recess 309 of the tibial bearing component 304 and a recess 322 (FIG. 4) of the tibial baseplate 302.

FIG. 4 shows an exploded view of the prosthesis assembly 300, the tibial baseplate 302, the tibial bearing component 304, the femoral component 306, the hinge post 308 and further illustrates a hinge axle 312, poly box 314, axle bushing 316, shackle 318 and a bushing .

The hinge post 308 is connected to femoral component 306 via the shackle 318, the axle bushing 316 and the hinge axle 312. A distal portion of the shackle 318 is received in the recess 309 in the tibial bearing component 304 and the distal portion is threaded or otherwise connected to the hinge post 308. The hinge post 308 extends distally through the recess 309 of the tibial bearing component 304 and is received in a recess 322 of the tibial baseplate 302. The recess 322 of the tibial baseplate 302 that receives the hinge post 308 can at least partially be formed by a keel 324 of the tibial baseplate 302. The hinge post 308 can be moveable, e.g., rotatable or capable of distraction, relative to the tibial bearing component 304 or the tibial baseplate 302. The hinge post 308 can be rotatably connected to femoral component 306 via the hinge axle 312. Thus, a longitudinal axis LA that defines a centerline of the hinge post 308 can define an axis of rotation/articulation ARA for the knee joint as the femoral component 306 and the tibial baseplate 302 are mechanically linked.

When assembled, the shackle 318 can be placed between opposing walls of poly box 314. When assembled on the hinge axle 312, the axle bushing 316 additionally resides within an aperture on a proximal portion of the shackle 318. The shackle 318 and hinge post 308 can be formed from suitable materials such as a titanium alloy, a cobalt-chromium alloy, or the like, while the axle bushing 316 and the poly box 314 can be formed from different materials such as plastic, e.g., UHMWPE. The axle bushing 316 can act as a bearing between the shackle 318 and the hinge axle 312. The poly box 314 can act as a bearing between the femoral component 306 and the shackle 318.

The prosthesis assembly 300 of FIG. 4 shows a system 326 of components where distraction of the knee is not limited by a capture element or other feature of the prosthesis assembly 300. Thus, the system 326 provides components that, when assembled, are configured for full distraction. Thus, soft tissue of the knee is relied upon to limit distraction between the femoral component 306 and the tibial baseplate 302 and the tibial bearing component 304 as discussed previously. The bushing 320 can be configured to insert into the recess 322 of at least the tibial baseplate 302. The bushing 320 can also insert into or through the recess 309 (FIG. 3) of the tibial bearing component 304 in some examples. The bushing 320 can be configured to receive at least a portion of the hinge post 308. The bushing 320 can act as a bearing between the hinge post 308 and the tibial baseplate 302. The hinge post 308 can be moveable generally proximal/distal relative to the bushing 320.

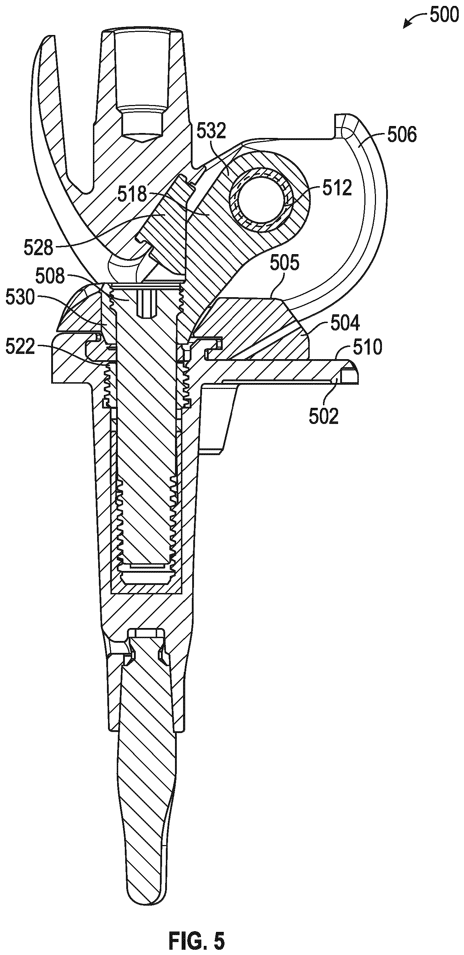

FIG. 5 is a cross-sectional view of an example of a prosthesis assembly for a constrained knee (prosthesis assembly 500), according to an example of the present application. The prosthesis assembly 500 includes a tibial tray 502, a tibial bearing component 504, a femoral component 506, a hinge post 508, a shackle 518, and a hinge axle 512.

The tibial tray 502, e.g., the tibial baseplate 302 from FIGS. 3-4, can extend between a proximal surface 510, e.g., the proximal surface 310 of FIG. 3, to a distal surface. The proximal surface 510 and the distal surface can define a periphery therebetween. The periphery can extend between an anterior edge and an posterior edge of the tibial tray 502. The proximal surface 510 can include a recess 522 that can extend toward the distal surface. The recess 522, e.g., the recess 322 from FIG. 4, can be configured to at least partially receive the hinge post 508.

The tibial bearing component 504, e.g., the tibial bearing component 304 from FIGS. 3-4, can be engageable with the tibial tray 502. For example, the tibial tray 502 can support the tibial bearing component 504 while the tibial bearing component 504 engages with the femoral component 506. The tibial bearing component 504 includes an articular surface 505. The articular surface 505 can be configured to engage with the femoral component 506. More specifically, the articular surface 505 can be configured to engage with the medial and lateral condyles of the femoral component 506.

The femoral component 506, e.g., the femoral component 306 from FIGS. 3-4, contacts the articular surface 505 of the tibial bearing component 504 and is configured to articulate with the tibial bearing component 504.

The hinge post 508, e.g., the hinge post 308 from FIGS. 3-4, extends through the tibial bearing component 504 and is at least partially received in a recess 522 of the tibial tray 502. The hinge post 508 can include a portion within the recess 522 of the tibial tray 502 and a portion extending outside the recess 522 of the tibial tray 502. The portion of the hinge post 508 extending within the recess 522 can include threads that are complementary to threads within the recess 522. The portion of the hinge post 508 that extends outside the recess 522 can be configured to extend through the femoral component 506 such that the femoral component 506 surrounds the hinge post 508 when the hinge post 508 is installed into the recess 522 of the tibial tray 502. The portion of the hinge post 508 that extends outside of the recess 522 can be received by a bushing that includes a threaded surface, any other attachment mechanism, or the like, that can be complementary to an attachment mechanism on the shackle 518.

The shackle 518, e.g., the shackle 318 from FIGS. 3 and 4, can be configured to connect to the hinge post 508 and have other components of the prosthesis assembly 500 attached thereto to attach the additional components to the prosthesis assembly 500. The shackle 518 can extend between a first-end portion 530 and a second-end portion 532. The first-end portion 530 can be configured to attach, couple, or mate with the hinge post 508. For example, the first-end portion 530 can include a threaded surface that is complementary to the threaded surface of the portion of the hinge post 508 that extends outside of the recess 522. The second-end portion 532 can be configured to receive the hinge axle 512 to attach one or more components of the prosthesis assembly 500 to the shackle 518. For example, the second-end portion 532 can include an aperture that is configured to receive the hinge axle 512. The shackle 518 can be configured to be inserted into the intercondylar region 507 of the femoral component 506.

The hinge axle 512, e.g., the hinge axle 312 from FIGS. 3 and 4, cis configured to secure the femoral component 506, among other components, to the shackle 518 and via the shackle 518 to the hinge post 508. The hinge axle 512 can be an elongated member that extends a distance greater than the thickness of the femoral component 506 so that the hinge axle 512 can be used to hold components, including the femoral component 506, onto the shackle 518. For example, the hinge axle 512 can attach the femoral component 506 and the walls 514 to the shackle 518 via the aperture in the shackle 518 that is configured to receive the hinge axle 512. The prosthesis assembly 500 can also include one or more bushings, washers, or any other component that can be complementary to the interactions between the hinge axle 512 and the aperture in the shackle 518.

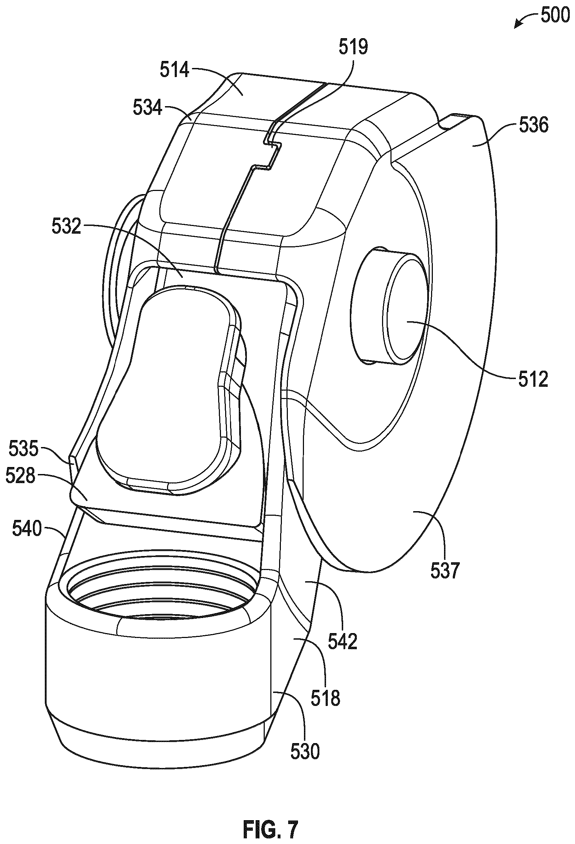

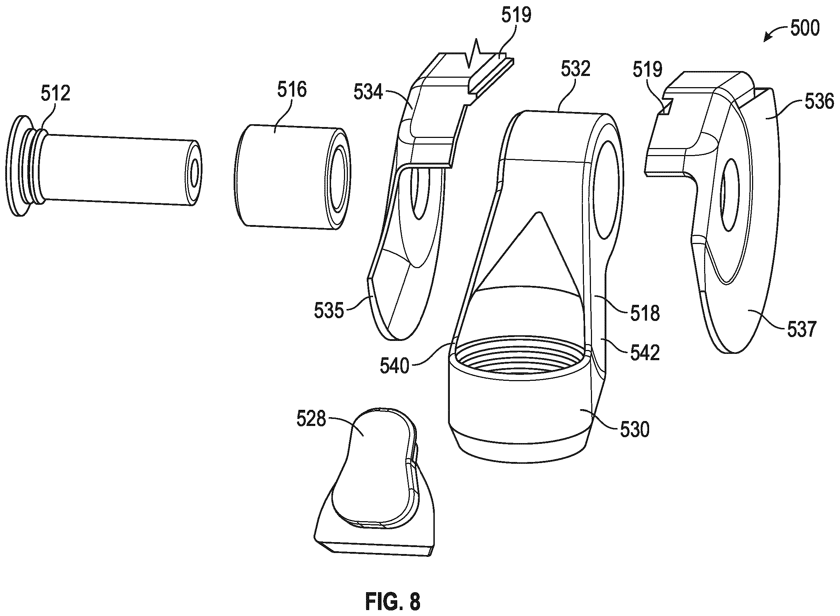

The walls 514, e.g., the poly box 314 from FIGS. 3-4, The walls 514 will be discussed in more detail below with reference to FIGS. 7 and 8.

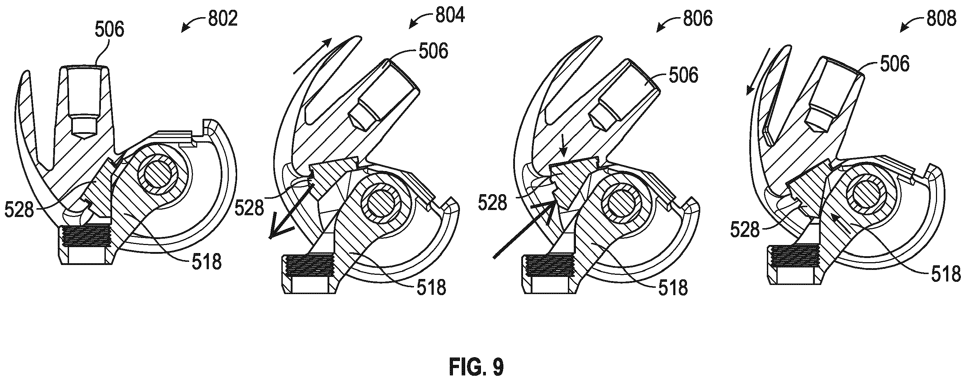

The prosthesis assembly 500 can also include a bump stop 528 that can be removably attached to the femoral component 506. As shown in FIG. 5, when the prosthesis assembly 500 is in an extended state, the bump stop 528 can contact the shackle 518 to prevent additional rotation of the femoral component 506 related to the tibial bearing component 504. The bump stop 528 will be discussed in more detail below with reference to FIGS. 7-9.

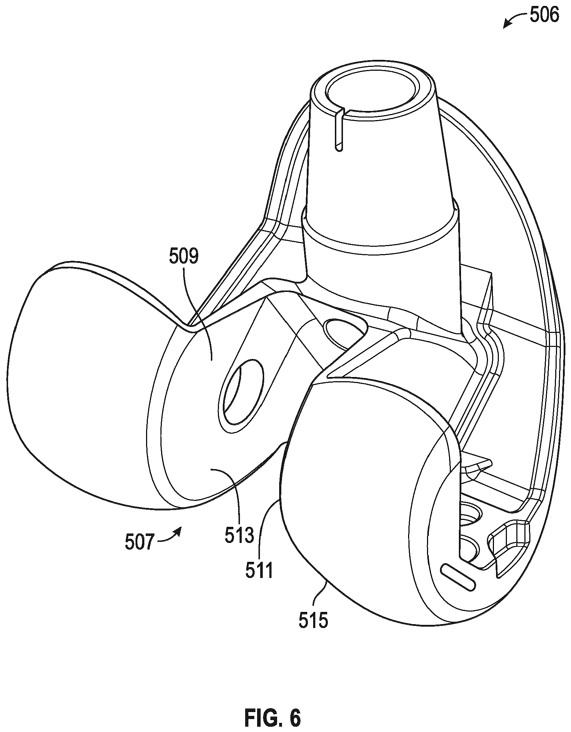

FIG. 6 is a perspective view of an example of a femoral component, e.g., the femoral component 506, according to an embodiment of the present application. In examples, the femoral component 506 can include an intercondylar region 507 that extends between the medial and lateral condyles of the femoral component 506. The femoral component 506 can include an inner medial condyle wall 509 and an inner lateral condyle wall 511. The intercondylar region 507 can extend between the inner medial condyle wall 509 and the inner lateral condyle wall 511. The intercondylar region 507, the inner medial condyle wall 509, and the inner lateral condyle wall 511 can be configured to engage with the shackle 518 and, more specifically, the walls 514 during articulation of the prosthesis assembly 500.

The inner medial condyle wall 509 can include a frustoconical profile 513, and the inner lateral condyle wall 511 can include a frustoconical profile 515. In one example, the frustoconical profile 513 can extend the entirety of the inner medial condyle wall 509. In another example, the frustoconical profile 513 can extend only a portion of the inner medial condyle wall 509. In one example, the frustoconical profile 515 can extend the entirety of the inner lateral condyle wall 511. In another example, the frustoconical profile 515 can extend only a portion of the inner lateral condyle wall 511. The frustoconical profile 513 of the inner medial condyle wall 509 and the frustoconical profile 515 of the inner lateral condyle wall 511 can maximize a thickness of the inner medial condyle wall 509 and the inner lateral condyle wall 511, respectively, to minimize a space that the inner medial condyle wall 509 and the inner lateral condyle wall 511 take up while maximizing the strength of the inner medial condyle wall 509 and the inner lateral condyle wall 511. Moreover, the frustoconical profile 513 and the frustoconical profile 515 can be configured to reduce stress concentrations in the inner medial condyle wall 509 and the inner lateral condyle wall 511, respectively.

FIGS. 7 and 8 will be discussed together below. FIG. 7 is a perspective view of a portion of a constrained knee prosthesis, e.g., the prosthesis assembly 500, according to an example of the present application. FIG. 8 is an exploded view of the prosthesis assembly 500. The prosthesis assembly 500 can include two or more poly walls or serviceable walls (walls 514). The walls 514 can be configured to at least surround a portion of the shackle 518. In examples, the shackle 518 can be an over-mold that is held in place over the shackle 518 by the hinge axle 512 extending through apertures of the walls 514. In another example, the walls 514 can fit over the shackle 518 such that the hinge axle 512 holds the walls 514 on the shackle 518. Here, the walls 514 can be attached to the shackle 518 via the hinge axle 512 and the aperture in the second-end portion 532 of the shackle 518.

As shown above in FIGS. 3-4, the poly box 314 can be a one-piece design configured to partially surround the shackle 318. However, as shown in FIGS. 7 and 8, the walls 514 are a two-piece design configured to at least partially surround the shackle 518. Thus, the walls 514 include a first wall 534 and a second wall 536. The two-piece design of the walls 514 makes the walls 514 easier to manufacture because the two-piece design of the walls 514 is less complex than the one-piece design of the poly box 314. For example, the first wall 534 and the second wall 536 can be manufactured separately and assembled before or during the assembly of the prosthesis assembly 500.

The first wall 534 is configured to cover a first wall 540 of the shackle 518. The second wall 536 is configured to cover a second wall 542 of the shackle 518. The first wall 534 and the second wall 536 can come together on an anterior edge 544 of the shackle 518. The first wall 534 and the second wall 536 include an attachment mechanism 519 that removably couples the first wall 534 to the second wall 536 to form walls 514. The attachment mechanism 519 can also restrain relative movement between the first wall 534 and the second wall 536 in one or more directions. For example, the attachment mechanism 519 can include a dovetail formation that couples the first wall 534 and the second wall 536. In another example, the attachment mechanism 519 can include a tongue and groove, which can couple the first wall 534 and the second wall 536. In yet another example, the attachment mechanism 519 can be any attachment mechanism that can couple the first wall 534 and the second wall 536 together. For example, the attachment mechanism 519 can include tabs, slots, patterns, protrusions, grooves, or any other geometric feature that can be used to couple the first wall 534 and the second wall 536, or the like.

At least a portion of the first wall 534 includes a frustoconical profile 535, and at least a portion of the second wall 536 can include a frustoconical profile 537. The frustoconical profile 535 of the first wall 534 can be complementary to the frustoconical profile 513 of the inner medial condyle wall 509, and the frustoconical profile 537 of the second wall 536 can be complementary to the frustoconical profile 515 of the inner lateral condyle wall 511. For example, during engagement of the prosthesis assembly 500, the frustoconical profile 513 of the inner medial condyle wall 509 can engage with the frustoconical profile 535 of the first wall 534 and the frustoconical profile 515 of the inner lateral condyle wall 511 can engage with the frustoconical profile 537 of the second wall 536. The frustoconical profiles, e.g., frustoconical profile 513, the frustoconical profile 515, the frustoconical profile 535, and the frustoconical profile 537, can provide additional clearance between the femoral component 506 and the walls 514 while maintaining the strength of the femoral component 506 and the walls 514 as compared to a stepped design or any other design that can be used to improve the clearance between the femoral component 506 and the shackle 518.

FIG. 8 is a cross-sectional view of a portion of the prosthesis assembly 500, according to an example of the present application. In FIG. 8, the prosthesis assembly 500 is shown in different orientations to illustrate how different components of the prosthesis assembly 500 interact with each other during the manipulation of the prosthesis assembly 500.

At orientation 802, a knee joint or the prosthesis assembly 500 can be extended such that the bump stop 528 contacts the shackle 518 to prevent the femoral component 506 from rotating further with relation to the tibial component, e.g., the tibial bearing component 504 from FIG. 5. For example, the bump stop 528 can be in contact with the shackle 518 to prevent further rotation (or further extension) of the prosthesis assembly 500. As shown in FIG. 8, a size of the bump stop 528 can determine how much rotation is permitted by the prosthesis assembly 500. For example, a height or thickness between a surface of the bump stop 528 that faces the femoral component 506 and a surface of the bump stop 528 that is configured to contact the shackle 518 can determine a limit of rotation of the femoral component 506 rotates relative to the tibial bearing component 504 (FIG. 5). For example, decreasing the thickness the bump stop 528 can permit more rotation before the bump stop 528 contacts the shackle 51. The thickness of the bump stop 528 can be increased to permit less rotation of the femoral component 506 with relation to the tibial bearing component 504. In another example, the shape of the surface of the bump stop 528 can be changed such as to change when the 528 contacts the shackle 518. For example, the 528 can be flat, rounded, or any other shape that can affect the amount of rotation of the femoral component 506 with relation to the tibial bearing component 504, or the like.

In examples, a system can include multiple of the bump stop 528 such as to provide multiple angles of rotation of the femoral component 506 in relation to the tibial bearing component 504. Here, the system can provide multiple of the bump stop 528 so that the bump stop 528 can be selected before or during the assembly of the prosthesis assembly 500 or after the implantation of the prosthesis assembly 500 to adjust the amount of rotation of the femoral component 506 in relation to the tibial bearing component 504 before the bump stop 528 contacts the shackle 518. For example, a surgeon can implant the prosthesis assembly 500 and determine the appropriate bump stop 528 based on range of motion tests, or any other indicator. In another example, the bump stop 528 can be changed while the knee of the patient is healing. For example, the bump stop 528 can initially be large to prevent more rotation of the femoral component 506 relative to the tibial bearing component 504, and as the patient heals, the bump stop 528 can be changed to a smaller size of the bump stop 528 to permit more rotation of the femoral component 506 relative to the tibial bearing component 504.

At orientation 804, the knee joint and the prosthesis assembly 500 can articulate to rotate the femoral component 506 about the hinge axle 512 to move the bump stop 528 away from the shackle 518. For example, the state of the prosthesis assembly 500 or the knee joint where the bump stop 528 is furthest away from the axle can be called the flexed state. In the flexed state, the bump stop 528 can be exposed or accessible from the front of the prosthesis assembly 500. In the flexed state, the bump stop 528 can be serviced. For example, the bump stop 528 can be removed or replaced with a bump stop of a different size. As can be seen in FIG. 8, the bump stop 538 can include a ridge, or a bulge, that can fit within a groove or a seat of the femoral component 506 such that the bump stop 538 can be pressed into the femoral component 506 to couple the bump stop 538 to the femoral component 506. Similarly, the bump stop 538 can be removed by passing the ridge or bulge of the bump stop 538 past the groove of the femoral component 506. In another example, any other press fit, or quick coupling and decoupling fitting can be used to secure the bump stop 538 within the femoral component 506.

In another example, the seating of the bump stop 528 within the femoral component 506 can be adjusted to change the amount of rotation of the femoral component 506 with relation to the tibial bearing component 504 before the bump stop 528 contacts the shackle 518. For example, the bump stop 528 can include multiple ridges or protrusions that can fit within grooves of the femoral component 506 to adjust the seating of the bump stop 528 and change a gap between the femoral component 506 and the shackle 518 when the femoral component 506 contacts the shackle 518. Such an adjustment can change an amount of rotation before the bump stop 528 contacts the shackle 518 to alter an amount of rotation of the femoral component 506 with relation to the tibial bearing component 504.

In the example shown in orientation 806, the bump stop 528 from orientations 802 and 804 can be removed and replaced with a bump stop, e.g., the bump stop 528 of a different size as shown in orientations 806 and 808. As shown in orientation 806, the bump stop 528 can be replaced with a larger version of the bump stop 528. As discussed above, the larger version of the bump stop 528 can contact the shackle 518 earlier to prevent rotation of the femoral component 506 in relation to the tibial bearing component 504. In another example, the size of the bump stop 528 can be decreased to increase the amount of rotation of the femoral component 506 with relation to the tibial bearing component 504.

As shown in orientation 808, an amount of rotation of the femoral component 506 with relation to the tibial bearing component 504 can be altered by installing a bump stop, e.g., the bump stop 528 of a different size shown in orientations 806 and 808. As shown in orientation 808, a bump stop 528 of increased size as compared to the bump stop 528 in orientations 802 and 804, can decrease the amount of rotation of the femoral component 506 with relation to the tibial bearing component 504.

The above-detailed description includes references to the accompanying drawings, which form a part of the detailed description. The drawings show, by way of illustration, specific embodiments that may be practiced. These embodiments are also referred to herein as "examples." Such examples may include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and those documents so referenced, the usage in the incorporated reference(s) should be considered supplementary to that of this document; for irreconcilable inconsistencies, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The term "about," as used herein, means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. In one aspect, the term "about" means plus or minus 10% of the numerical value of the number with which it is being used. Therefore, about 50% means in the range of 45%-55%. Numerical ranges recited herein by endpoints include all numbers and fractions subsumed within that range, e.g., 1 to 5 includes 1, 1.5, 2, 2.75, 3, 3.90, 4, 4.24, and 5. Similarly, numerical ranges recited herein by endpoints include subranges subsumed within that range, e.g., 1 to 5 includes 1-1.5, 1.5-2, 2-2.75, 2.75-3, 3-3.90, 3.90-4, 4-4.24, 4.24-5, 2-5, 3-5, 1-4, and 2-4. It is also to be understood that all numbers and fractions thereof are presumed to be modified by the term "about."

The above description is intended to be illustrative, and not restrictive. For example, the above-described examples (or one or more aspects thereof) may be used in combination with each other. Other embodiments may be used, such as by one of ordinary skill in the art upon reviewing the above description. The Abstract is to allow the reader to quickly ascertain the nature of the technical disclosure and is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description, with each claim standing on its own as a separate embodiment. The scope of the embodiments should be determined with reference to the appended claims, along with the full scope of equivalents to which such claims are entitled.

## Claims

1. A prosthesis assembly (500) for a constrained knee comprising:
a tibial tray (502);
a tibial bearing component (504) including an articular surface (505);
a hinge post (508) extending through the tibial bearing component (504) and at least partially received in a recess (522) of the tibial tray (502);
a femoral component (506) that contacts the articular surface (505) of the tibial bearing component (504) and is configured to articulate with the tibial bearing component (504);
a shackle (518) coupled to the hinge post (508) at a first end portion, the shackle (518) configured to be inserted between a medial condyle and a lateral condyle of the femoral component (506), the shackle (518) including:
a first side; and
a second side opposite the first side;
walls (514) positioned between the femoral component (506) and the shackle (518) and engaged by the femoral component (506), the walls including:
a first wall (534) configured to cover the first side of the shackle (518), an outer surface of the first wall (534) at least partially includes a first frustoconical profile; and
a second wall (536) configured to cover the second side of the shackle (518), the first wall (534) removably attached to the second wall (536); and
a hinge axle (512) configured to secure the femoral component (506) to the shackle (518).

2. The prosthesis assembly (500) of claim 1, comprising:
a bump stop removably attached to the femoral component (506), the bump stop configured to contact the shackle (518) at a set limit of rotation of the femoral component (506) relative to the tibial bearing component (504).

3. The prosthesis assembly (500) of claim 2, wherein the bump stop is configured to be removed when a knee joint is in a flexion state.

4. The prosthesis assembly (500) of any of claims 2-3, wherein a size of the bump stop determines the set limit of rotation of the femoral component (506) with relation to the tibial bearing component (504).

5. The prosthesis assembly (500) of claim 4, wherein the bump stop is configured to be selectively serviceable.

6. The prosthesis assembly (500) of any of claims 1-5, wherein the first wall (534) and the second wall (536) are coupled by an engagement feature.

7. The prosthesis assembly (500) of claim 6, wherein the engagement feature includes a dovetail connection between the first wall (534) and the second wall (536).

8. The prosthesis assembly (500) of any of claims 1-7, wherein an outer surface of the second wall (536) at least partially includes a second frustoconical profile.

9. The prosthesis assembly (500) of claim 8, wherein an inner wall of the medial condyle of the femoral component (506) includes a third frustoconical profile complementary to the first frustoconical profile.

10. The prosthesis assembly (500) of claim 10, wherein an inner wall of the lateral condyle of the femoral component (506) includes a fourth frustoconical profile that is complementary to the second frustoconical profile.

## Patentansprüche

1. Prothesenbaugruppe (500) für ein eingeschränktes Knie, umfassend:
einen Tibiaträger (502);
eine Tibialagerkomponente (504), die eine Gelenkoberfläche (505) einschließt;
einen Scharnierpfosten (508), der sich durch die Tibialagerkomponente (504) erstreckt und mindestens teilweise in einer Aussparung (522) des Tibiaträgers (502) aufgenommen ist;
eine Femurkomponente (506), die die Gelenkoberfläche (505) der Tibialagerkomponente (504) kontaktiert und dazu konfiguriert ist, mit der Tibialagerkomponente (504) ein Gelenk zu bilden;
einen Bügel (518), der an einem ersten Endabschnitt an den Scharnierpfosten (508) gekoppelt ist, wobei der Bügel (518) dazu konfiguriert ist, zwischen einem medialen Kondylus und einem lateralen Kondylus der Femurkomponente (506) eingeführt zu werden, wobei der Bügel (518) Folgendes einschließt:
eine erste Seite; und
eine zweite Seite gegenüber der ersten Seite;
Wände (514), die zwischen der Femurkomponente (506) und dem Bügel (518) positioniert sind und durch die Femurkomponente (506) in Eingriff genommen werden, wobei die Wände Folgendes einschließen:
eine erste Wand (534), die dazu konfiguriert ist, die erste Seite des Bügels (518) zu bedecken, wobei eine Außenoberfläche der ersten Wand (534) mindestens teilweise ein erstes kegelstumpfförmiges Profil einschließt; und
eine zweite Wand (536), die dazu konfiguriert ist, die zweite Seite des Bügels (518) zu bedecken, wobei die erste Wand (534) entfernbar an der zweiten Wand (536) angebracht ist; und
eine Scharnierachse (512), die dazu konfiguriert ist, die Femurkomponente (506) an dem Bügel (518) zu sichern.

2. Prothesenbaugruppe (500) nach Anspruch 1, umfassend:
einen Anschlagpuffer, der entfernbar an der Femurkomponente (506) angebracht ist, wobei der Anschlagpuffer dazu konfiguriert ist, den Bügel (518) an einer festgelegten Drehgrenze der Femurkomponente (506) relativ zu der Tibialagerkomponente (504) zu kontaktieren.

3. Prothesenbaugruppe (500) nach Anspruch 2, wobei der Anschlagpuffer dazu konfiguriert ist, entfernt zu werden, wenn sich ein Kniegelenk in einem Flexionszustand befindet.

4. Prothesenbaugruppe (500) nach einem der Ansprüche 2-3, wobei eine Größe des Anschlagpuffers die festgelegte Drehgrenze der Femurkomponente (506) in Bezug auf die Tibialagerkomponente (504) bestimmt.

5. Prothesenbaugruppe (500) nach Anspruch 4, wobei der Anschlagpuffer dazu konfiguriert ist, selektiv wartbar zu sein.

6. Prothesenbaugruppe (500) nach einem der Ansprüche 1-5, wobei die erste Wand (534) und die zweite Wand (536) durch ein Eingriffsmerkmal gekoppelt sind.

7. Prothesenbaugruppe (500) nach Anspruch 6, wobei das Eingriffsmerkmal eine Schwalbenschwanzverbindung zwischen der ersten Wand (534) und der zweiten Wand (536) einschließt.

8. Prothesenbaugruppe (500) nach einem der Ansprüche 1-7, wobei eine Außenoberfläche der zweiten Wand (536) mindestens teilweise ein zweites kegelstumpfförmiges Profil einschließt.

9. Prothesenbaugruppe (500) nach Anspruch 8, wobei eine Innenwand des medialen Kondylus der Femurkomponente (506) ein drittes kegelstumpfförmiges Profil einschließt, das zu dem ersten kegelstumpfförmigen Profil komplementär ist.

10. Prothesenbaugruppe (500) nach Anspruch 10, wobei eine Innenwand des lateralen Kondylus der Femurkomponente (506) ein viertes kegelstumpfförmiges Profil einschließt, das zu dem zweiten kegelstumpfförmigen Profil komplementär ist.

## Revendications

1. Ensemble prothèse (500) destiné à un genou contraint comprenant :
un plateau tibial (502) ;
un élément de support tibial (504) comprenant une surface articulaire (505) ;
un montant d'articulation (508) s'étendant à travers l'élément de support tibial (504) et au moins partiellement reçu dans un évidement (522) du plateau tibial (502) ;
un élément fémoral (506) qui est en contact avec la surface articulaire (505) de l'élément de support tibial (504) et est conçu pour s'articuler avec l'élément de support tibial (504) ;
une manille (518) couplée au montant d'articulation (508) au niveau d'une première partie d'extrémité, la manille (518) étant conçue pour être insérée entre un condyle médial et un condyle latéral de l'élément fémoral (506), la manille (518) comprenant :
un premier côté ; et
un second côté opposé au premier côté ;
des parois (514) positionnées entre l'élément fémoral (506) et la manille (518) et mises en prise par l'élément fémoral (506), les parois comprenant :
une première paroi (534) conçue pour recouvrir le premier côté de la manille (518), une surface externe de la première paroi (534) comprenant au moins partiellement un premier profil tronconique ; et
une seconde paroi (536) conçue pour recouvrir le second côté de la manille (518), la première paroi (534) étant fixée de manière amovible à la seconde paroi (536) ; et
un axe d'articulation (512) conçu pour fixer l'élément fémoral (506) à la manille (518).

2. Ensemble prothèse (500) selon la revendication 1, comprenant :
une butée d'amortissement fixée de manière amovible à l'élément fémoral (506), la butée d'amortissement étant conçue pour entrer en contact avec la manille (518) à une limite de rotation définie de l'élément fémoral (506) par rapport à l'élément de support tibial (504).

3. Ensemble prothèse (500) selon la revendication 2, ladite butée d'amortissement étant conçue pour être retirée lorsqu'une articulation du genou se trouve dans un état de flexion.

4. Ensemble prothèse (500) selon l'une quelconque des revendications 2 à 3, une taille de la butée d'amortissement déterminant la limite de rotation définie de l'élément fémoral (506) par rapport à l'élément de support tibial (504).

5. Ensemble prothèse (500) selon la revendication 4, ladite butée d'amortissement étant conçue pour être réparable de manière sélective.

6. Ensemble prothèse (500) selon l'une quelconque des revendications 1 à 5, ladite première paroi (534) et ladite seconde paroi (536) étant couplées par un élément de mise en prise.

7. Ensemble prothèse (500) selon la revendication 6, ledit élément de mise en prise comprenant un raccord en queue d'aronde entre la première paroi (534) et la seconde paroi (536).

8. Ensemble prothèse (500) selon l'une quelconque des revendications 1 à 7, une surface externe de la seconde paroi (536) comprenant au moins partiellement un deuxième profil tronconique.

9. Ensemble prothèse (500) selon la revendication 8, une paroi interne du condyle médial de l'élément fémoral (506) comprenant un troisième profil tronconique complémentaire au premier profil tronconique.

10. Ensemble prothèse (500) selon la revendication 10, une paroi interne du condyle latéral de l'élément fémoral (506) comprenant un quatrième profil tronconique qui est complémentaire au deuxième profil tronconique.
